(19) Europäisches Patentamt
European Patent Office
Office européen des brevets

(11) **EP 2 108 726 A2**

(12) **EUROPÄISCHE PATENTANMELDUNG**

(43) Veröffentlichungstag:
**14.10.2009 Patentblatt 2009/42**

(51) Int Cl.:
*D06F 33/02* (2006.01)    *D06F 39/00* (2006.01)

(21) Anmeldenummer: **09008439.3**

(22) Anmeldetag: **29.06.2009**

(84) Benannte Vertragsstaaten:
**AT BE BG CH CY CZ DE DK EE ES FI FR GB GR HR HU IE IS IT LI LT LU LV MC MK MT NL NO PL PT RO SE SI SK TR**

(71) Anmelder: **V-Zug AG**
**CH-6301 Zug (CH)**

(72) Erfinder:
• **Machau, Susanne**
**6330 Cham (CH)**

• **Rohr, Hansjörg**
**8910 Affoltern a. A. (CH)**

(74) Vertreter: **Sutter, Kurt et al**
**E. Blum & Co**
**Patentanwälte VSP**
**Vorderberg 11**
**8044 Zürich (CH)**

(54) **Waschmaschine mit Hygieneanzeige**

(57)    In einer Waschmaschine wird mindestens einem Teil der Betriebsprogramme ein Hygienerisikowert zugeordnet. Aus den Hygienerisikowerten der zuletzt durchgeführten Betriebsprogramme wird ein Hygienebedarfswert ermittelt. Abhängig vom Bedarfswert wird eine Hygieneanzeige des Geräts aktiviert oder deaktiviert, die dem Benutzer anzeigt, ob eine Entkeimung des Geräts mittels eines Entkeimungsprogramms bzw. eines Programms mit einer Waschtemperatur von ≥ 60°C ratsam ist.

**Fig. 1**

**Beschreibung**

Gebiet der Erfindung

Die Erfindung betrifft eine Waschmaschine sowie ein Verfahren zum Betrieb einer Waschmaschine gemäss Oberbegriff der unabhängigen Ansprüche.

Hintergrund

Waschmaschinen sind in der Regel mit einer Steuereinheit ausgestattet, welche zur Durchführung verschiedener Betriebsprogramme ausgestaltet ist.

Es ist bekannt, mindestens eines dieser Betriebsprogramme als Entkeimungs- bzw. Hygieneprogramm auszugestalten, um die Trommel bzw. den Waschraum bei einer Temperatur von mindestens 60°C zu entkeimen. Ein derartiges Hygieneprogramm ist in DE 197 51 028 beschrieben. Solche Programme gewinnen an Bedeutung, da heute vermehrt bei tiefen Temperaturen gewaschen wird

Aus EP 808 936 ist es bekannt, die Waschmaschine mit einer Hygieneanzeige auszustatten, welche aktiviert wird, wenn das zuletzt durchgeführte Betriebsprogramm unterhalb 60°C lag. Auf diese Weise wird dem Benutzer angezeigt, dass die Hygiene des Geräts unter Umständen mangelhaft und die Durchführung des Hygieneprogramms ratsam ist.

Von Nachteil bei bekannten Maschinen ist der erhöhte Energie- und Zeitbedarf für die Durchführung des Hygieneprogramms.

Darstellung der Erfindung

Es stellt sich die Aufgabe, eine Waschmaschine bereitzustellen, welche ebenfalls ein Hygieneprogramm bzw. Entkeimungsprogramm und eine Hygieneanzeige besitzt, bei welcher jedoch der Energie- und Zeitbedarf reduziert ist.

Diese Aufgabe wird von der Waschmaschine und dem Verfahren gemäss den unabhängigen Ansprüchen gelöst.

Demgemäss ist die Steuerung der Waschmaschine so ausgestaltet, dass sie mindestens einem Teil der Betriebsprogramme mindestens teilweise unterschiedliche Hygienerisikowerte zuordnet und dass sie die Hygienerisikowerte einer Zahl N zuletzt durchgeführter Betriebsprogramme zu einem Hygienebedarfswert kumuliert werden. Dabei ist die Zahl N mindestens zeitweise grösser als 1. Abhängig vom Hygienebedarfswert aktiviert oder deaktiviert die Steuereinheit die Hygieneanzeige. Somit wird also die Hygieneanzeige abhängig von der "Waschgeschichte" der Maschine über die letzten Betriebsprogramme (d.h. in der Regel über mehrere Betriebsprogramme) gesteuert, wobei nicht allen Betriebsprogrammen der gleiche Hygienerisikowert zugeordnet ist. Dies erlaubt es, eine differenzierte Risikobeurteilung aufgrund der Waschgeschichte durchzuführen, bevor entschieden wird, ob die Hygieneanzeige aktiviert werden soll.

Anspruchsgemäss werden die Hygienerisikowerte einer Zahl N zuletzt durchgeführter Betriebsprogramme zu einem Hygienebedarfswert "kumuliert". Unter "kumulieren" ist dabei die Ermittlung des Hygienebedarfswerts in Abhängigkeit von den Hygienerisikowerten $R(p_i)$ der N durchlaufenen Betriebprogramme $p_i$ zu verstehen. Dies kann z.B. so ausgedrückt werden, dass, mathematisch gesprochen, die Entscheidung zur Aktivierung abhängig vom Wert einer Funktion

$$H(R(p_1), \dots R(p_N))$$

erfolgt, welche ihrerseits abhängig von den Hygienerisikowerten $R(p_i)$ (i = 1 ... N) der N zuletzt durchgeführten Betriebsprogramme $p_1 \dots p_N$ ist.

Das anspruchsgemässe Entkeimungsprogramm kann ein speziell für diesen Zweck vorgesehenes Programm sein, das in der Steuereinheit abgelegt ist, oder es kann sich um eines der normalen Waschprogramme mit einer Temperatur von mindestens 60°C handeln.

Kurze Beschreibung der Zeichnung

Weitere bevorzugte Ausführungen ergeben sich aus den abhängigen Ansprüchen sowie aus der nun folgenden Beschreibung anhand der Figur. Die Figur zeigt ein Funktionsschaltbild einer Waschmaschine.

Wege zur Ausführung der Erfindung

Die Waschmaschine 1 besitzt eine Trommel 2 zur Aufnahme der zu reinigenden Wäsche sowie eine Einund Ausgabeeinheit 3 mit Eingabeelementen 4 und Anzeigelementen 5. Die Funktionen der Waschmaschine 1 werden von einer Steuereinheit 6 gesteuert.

Die Steuereinheit 6 ist dazu ausgestaltet, die Waschmaschine 1 gemäss einem von mehreren Betriebprogrammen zu steuern. Die Betriebsprogramme sind in der Form von Tabellen und/oder Programmschritten in der Steuereinheit 6 abgespeichert. Sie unterscheiden sich voneinander z.B. aufgrund ihrer Waschtemperatur (es werden z.B. Programme mit Temperaturen von 20°C, 30°C, 40°C, 60°C und 90°C zur Verfügung gestellt), der durchlaufenen Waschgänge (mit/ ohne Vorwaschen, mit/ohne Schleudern) oder verschiedener weiterer Betriebsparameter (z.B. Wassermenge, Schleuderzahl, etc.). Entsprechende Programme sind dem Fachmann bekannt.

Mindestens eines der Betriebsprogramme ist als Entkeimungsprogramm (Hygieneprogramm) ausgestaltet, bei welchem die Temperatur der Trommel 2 mittels Wasser und/oder Dampf auf mindestens 60°C erhöht wird. Entsprechende Entkeimungsprogramme sind z.B. aus EP 808 936 oder DE 197 51 028 bekannt. Das Einkeimungsprogramm ist vorzugsweise für den Benutzer als solches gekennzeichnet, es ist aber auch denkbar, eines der "normalen" Waschprogramme mit einer Temperatur von mindestens 60°C als Entkeimungsprogramm zu verwenden. In diesem Fall kann der Benutzer ein 60°C-Programm (mit oder ohne Wäsche) zur Entkeimung des Geräts einsetzen.

Der Benutzer kann durch Manipulation der Eingabeelemente 4 in bekannter Weise das gewünschte Betriebsprogramm anwählen.

Eines der Anzeigeelemente 5 wird im Folgenden als Hygieneanzeige 5a bezeichnet. Sie dient dazu, dem Benutzer anzuzeigen, dass die Durchführung des Entkeimungsprogramms ratsam ist. Die Hygieneanzeige 5a wird von der Steuerung 6 aktiviert oder deaktiviert. Dies kann abhängig von verschiedenen Parametern der durchlaufenen Betriebsprogramme geschehen. Im Folgenden werden verschiedene Möglichkeiten hierzu beschrieben.

Berücksichtigung der Temperatur

In einer ersten Ausführung sind den Betriebsprogrammen abhängig von ihrer Temperatur unterschiedliche Hygienerisikowerte R zugeordnet. Vorzugsweise steigt der Hygienerisikowert R mit abnehmender Temperatur der Betriebsprogramme, d.h. Betriebsprogrammen mit tiefer Temperatur wird bei der Berechnung des Hygienebedarfswerts grösseres Gewicht gegeben. Beispielsweise können, abhängig von den Temperaturen der Betriebsprogramme, folgende Hygienerisikowerte R verwendet werden:

| Temperatur | R |
|---|---|
| 20°C | 5 |
| 30°C | 4 |
| 40°C | 3 |
| 50°C | 2 |

Die Steuerung 6 errechnet die Summe der Risikowerte $R(p_i)$ der zuletzt durchgeführten Betriebsprogramme $p_1$, $p_2$, etc., d.h. die berechnet Funktion

$$H(p_1, \ldots, p_N) = R(p_1) + R(p_2) + \ldots$$

wobei $p_1$ das zuletzt durchgeführte Betriebsprogramm, $p_2$ das zuvor durchgeführte Betriebsprogramm, etc. kennzeichnet. Die Zahl N der berücksichtigen Betriebsprogramme kann dabei grundsätzlich beliebig hoch sein.

N ist mit Vorteil nicht grösser als die Zahl (vorzugsweise gleich der Zahl) der Betriebsprogramme, die durchgeführt wurden, nachdem das letzte Mal ein Betriebsprogramm mit einer Temperatur von mindestens 60°C durchlaufen wurde. Mit anderen Worten wird die Summierung also zurückgesetzt, sobald ein Betriebsprogramm mit einer Temperatur von mindestens 60°C durchgeführt wird.

Der Wert der Funktion H wird als Hygienebedarfswert bezeichnet und mit einem Schwellwert S verglichen. Solange der Schwellwert S nicht erreicht ist, bleibt die Hygieneanzeige 5a deaktiviert. Sobald der Schwellwert erreicht oder überschritten wurde, wird die Hygieneanzeige 5a von der Steuereinheit 6 aktiviert.

Bei Verwendung des oben genannten Beispiels für Zahlenwerte von R kann die Schwelle z.B. als S = 100 gewählt werden. Somit wird die Hygieneanzeige 5a aktiviert, wenn nacheinander 20 Betriebsprogramme mit einer Temperatur von 20°C durchgeführt wurden. Länger dauert es, wenn Betriebsprogramme höherer Temperaturen eingesetzt werden. Wird ein Betriebsprogramm mit einer Temperatur von 60°C oder mehr eingesetzt, oder wird das Entkeimungsprogramm aktiviert, so wird die Summe zurückgesetzt.

Zu beachten ist, dass die Risikowerte R als positive oder negative Zahlen eingesetzt werden können, wobei bei Verwendung negativer Zahlen auf ein Unterschreiten des Schwellwerts S geprüft wird.

Zudem kann die Summe zu einem Grundoffset addiert werden. Beispielsweise kann dieser Grundoffset z.B. 100 betragen und die Risikowerte R können geeignete negative Zahlen sein, in welchem Fall der Schwellwert z.B. bei 0 liegen kann.

Berücksichtigung der Standzeit:

Die Kumulierung der Risikowerte kann abhängig von der Standzeit $t_0$ der Maschine sein. Dieser Massnahme steht der Gedanke zugrunde, dass sich Keime bei längerer Standzeit der Maschine vermehren können. Insbesondere kommt es bei längerer Standzeit der Maschine zur Bildung von Biofilmen, besonders an feuchten Oberflächen.

Unter Standzeit ist dabei die Zeit zu verstehen, während welcher die Maschine zwischen zwei Betriebsprogrammen nicht benutzt wird.

Vorzugsweise hängt die Bewertung der Standzeit davon ab, ob die Türe der Waschmaschine während der Standzeit offen oder geschlossen war, da die Vermehrungsraten von Keimen bei offener und geschlossener Türe unterschiedlich sind. In der Regel vermehren sich Keime wegen der hohen Luftfeuchtigkeit bei geschlossener Türe besser, weshalb die Standzeit $t_0$ derart in den Hygienebedarfswert einfliessen sollte, dass der Schwellwert S früher erreicht wird, wenn die Türe während der Standzeit $t_0$ geschlossen war.

Im Rahmen des in obiger Tabelle gezeigten numerischen Beispiels können z.B.

- bei offener Türe ab der dritten Woche Standzeit pro Woche Standzeit 10 Punkte zum Hygienebedarfswert hinzugezählt werden;
- bei geschlossener Türe ab der fünften Woche Standzeit pro Woche Standzeit 5 Punkte zum Hygienebedarfswert hinzugezählt werden.

Um die Standzeit $t_0$ zu bestimmen, ist die Steuereinheit 6 vorzugsweise mit einer Echtzeituhr ausgerüstet.

Berücksichtigung des Waschmittels:

Wenn die Steuereinheit weiss, was für ein Waschmittel bei einem bestimmten Betriebsprogramm eingesetzt wird, so kann die Hygienebedarfsabschätzung weiter verbessert werden. Insbesondere in folgenden Fällen kann die Steuereinheit wissen, was für ein Waschmittel zum Einsatz kommt:

- Die Waschmaschine ist so ausgestaltet, dass die Steuereinheit die Waschmittel selbst auswählt und zugibt.
- Der Benutzer gibt über die Eingabeelemente 4 ein, was für ein Waschmittel zum Einsatz kommt.
- Die Waschmaschine ist dazu ausgestaltet, einen Produktcode auf der Verpackung des Waschmittels auszulesen.
- Die Waschmaschine ist mit mindestens einem Sensor versehen, der in der Lage ist, eine oder mehrere Komponenten des Waschmittels selbsttätig zu bestimmen.

Ist der Steuereinheit 6 mindestens ein Parameter des Waschmittels bekannt, so kann der Hygienerisikowert für ein Betriebsprogramm davon abhängen, was für ein Waschmittel (bzw. welche Waschmittel) bei dessen Durchführung eingesetzt wurde(n). Somit kann berücksichtigt werden, dass unterschiedliche Waschmittel unterschiedlichen Einfluss auf das Keimwachstum besitzen.

Insbesondere besitzen Bleichmittel eine abtötende oder zumindest hemmende Wirkung auf Keime. Deshalb wird der Hygienerisikowert für ein Betriebsprogramm unter Einsatz von Bleichmitteln bei der Berechnung des Hygienebedarfswerts schwächer gewichtet als für das gleiche Betriebsprogramm ohne Einsatz von Bleichmitteln, d.h. der Betrag des Hygienerisikowerts wird bei Einsatz eines Bleichmittels reduziert.

Berechnungsvarianten:

Im obigen Bespiel ist die Funktion H als Summe der Risikowerte R der einzelnen Betriebsprogramme $p_1$, ..., $p_N$ formuliert. Generell kann die Funktion $H = H(R(p_1), ... R(p_N))$ auch eine andere Abhängigkeit von den Risikowerten $R(p_i)$ besitzen.

Grundsätzlich liegt der Funktion H ein Modell zur Vermehrung der Keime zugrunde, welches berücksichtigt, dass die Keime sich abhängig davon vermehren, was für Betriebsprogramme die Waschmaschine durchläuft.

So kann z.B. berücksichtigt werden, dass ein Betriebsprogramm mit einer Temperatur von 50°C bei ausreichender Betriebsdauer durchaus in der Lage sein kann, einen gewissen Prozentsatz der Keime abzutöten, und dass ein Betriebsprogramm von 60°C aber sehr kurzer Dauer nicht alle Keime abtötet, sondern lediglich wiederum einen gewissen Prozentsatz davon.

Solchen Betriebsprogrammen, die die Keime teilweise abtöten, kann z.B. ein Hygienerisikowert mit negativem Vorzeichen zugeordnet werden, falls den Betriebsprogrammen, welche die Keime fördern, Hygienerisikowerte mit positivem Vorzeichen zugeordnet sind.

Denkbar ist es weiter, dass nicht eine Summation, sondern eine Produktbildung zur Berechnung der Funktion H eingesetzt wird. Möglich sind aber auch komplexere mathematische Abhängigkeiten, oder die Funktion H kann mittels eines nicht analytisch darstellbarem Algorithmus berechnet werden.

In einigen Beispielen wurde erwähnt, dass Hygienerisikowerte bei der Berechnung des Hygienebedarfswerts abhängig von gewissen Parametern (wie z.B. Temperatur oder Bleichmitteleinsatz) stärker oder weniger stark gewichtet werden. Darunter ist jeweils eine Massnahme zu verstehen, welche bewirkt, dass der Einfluss des Hygienerisikowerts auf den Hygienebedarfswert stärker bzw. schwächer wird, so dass der Schwellwert schneller bzw. langsamer erreicht wird. Falls z.B. der Hygienebedarfswert als Summe oder Produkt der (positiven) Hygienerisikowerte berechnet wird, ist ein Hygienerisikowert mit grossen numerischen Wert stärker gewichtet als ein Hygienerisikowert mit kleinem numerischen Wert.

Entkeimungsprogramm:

Wie bereits erwähnt, handelt es sich beim Entkeimungsprogramm um ein Betriebsprogramm, bei welchem die Temperatur der Trommel 2 auf mindestens 60°C erhöht wird. Dies kann mittels Wasser geschehen, das der Trommel 2 zugeführt und auf ausreichende Temperatur erwärmt wird.

Es kann jedoch auch mit Dampf erfolgen, wie es z.B. in EP 1 275 767 beschrieben wird. Dort wird eine Heizung am Bottich so betrieben, dass das sich im Bottich befindliche Wasser auf mindestens 70°C erhitzt wird, um so Dampf zu generieren.

Die Waschmaschine 1 kann jedoch auch mit einem Dampfgenerator 7 ausserhalb des Bottichs ausgerüstet sein, mit welchem Dampf mittels einer separaten Heizung (d.h. einer Heizung zusätzlich zur Bottichheizung) erzeugt und sodann in den Bottich eingeführt wird.

Bemerkungen:

Im obigen Beispiel nimmt der Hygienerisikowert mit abnehmender Temperatur des Betriebsprogramms zu. Ja nach zugrunde liegendem Simulationsmodell kann es jedoch auch sinnvoll sein, Betriebsprogrammen mit Temperaturen im Bereich 30 bis 40°C höhere Hygienerisikowerte zuzuordnen als entsprechenden Programmen mit anderen Temperaturen, da sich gewisse Keime bei Temperaturen zwischen 30 bis 40°C besonders gut vermehren.

Die Erfindung betrifft auch ein Verfahren zur Steuerung der beschriebenen Waschmaschine. Das Verfahren umfasst dabei einen oder mehrere der Schritte, welche die beschriebene Steuereinheit 6 durchführt. Alle abhängigen Ansprüche können grundsätzlich auch als abhängige Ansprüche des Verfahrens formuliert sein.

Die vorliegende Erfindung erlaubt eine differenzierte Aktivierung der Hygieneanzeige abhängig von der "Geschichte" des Geräts, so dass der Benutzer gezielt dann zum Durchführen des Entkeimungsprogramms aufgefordert werden kann, wenn ein solches auch tatsächlich sinnvoll ist.

**Patentansprüche**

1.  Waschmaschine mit
    einer Trommel (2) zur Aufnahme von zu waschenden Wäschestücken,
    einer Steuereinheit (6), welche zur Durchführung verschiedener Betriebsprogramme ausgestaltet ist, wobei mindestens ein Betriebsprogramm ein Entkeimungsprogramm ist, bei welchem die Trommel (2) mit Wasser oder Dampf von mindestens 60°C entkeimt wird,
    einer Eingabeeinheit (4) zur Auswahl eines gewünschten Betriebsprogramms durch den Benutzer,
    einer Hygieneanzeige (5a), welche von der Steuereinheit (6) angesteuert ist, um dem Benutzer die Ratsamkeit der Durchführung des Einkeimungsprogramms anzuzeigen,
    **dadurch gekennzeichnet, dass** die Steuereinheit (6) dazu ausgestaltet ist, mindestens einem Teil der Betriebsprogramme mindestens teilweise unterschiedliche Hygienerisikowerte zuzuordnen, die Hygienerisikowerte einer Zahl N zuletzt durchgeführter Betriebsprogramme zu einem Hygienebedarfswert H zu kumulieren und abhängig vom Hygienebedarfswert die Hygieneanzeige (5a) zu aktivieren oder deaktivieren, und dass die Zahl N mindestens zeitweise grösser als 1 ist.

**2.** Waschmaschine nach Anspruch 1, wobei die Steuereinheit (6) dazu ausgestaltet ist, eine Entscheidung zur Aktivierung der Hygieneanzeige (5a) abhängig vom Wert einer Funktion

$$H(R(p_1), \dots R(p_N))$$

zu fällen, wobei $p_1 \dots p_N$ die zuletzt durchgeführten N Betriebsprogramme und $R(p_i)$ die Hygienerisikowerte derselben sind.

**3.** Waschmaschine nach Anspruch 2, wobei die Steuereinheit (6) die Summe der Hygienerisikowerte $R(p_1)$, ... $R(p_N)$ errechnet und mit einem Schwellwert vergleicht.

**4.** Waschmaschine nach einem der Ansprüche 2 bis 4, wobei die Funktion H zudem abhängig ist von einer Standzeit $t_0$ der Maschine.

**5.** Waschmaschine nach Anspruch 4, wobei die Funktion H abhängig ist von der Standzeit $t_0$ seit Durchführung des letzten Betriebsprogramms mit einer Temperatur von mindestens 60°C.

**6.** Waschmaschine nach einem der Ansprüche 4 oder 5, wobei die Funktion H davon abhängt, ob eine Türe der Waschmaschine während der Standzeit $t_0$ offen oder geschlossen war.

**7.** Waschmaschine nach Anspruch 6, wobei die Standzeit $t_0$ derart in den Hygienebedarfswert einfliesst, dass der Schwellwert früher erreicht wird, wenn die Türe während der Standzeit t0 geschlossen war.

**8.** Waschmaschine nach einem der Ansprüche 2 bis 7, wobei bei der Berechnung des Hygienebedarfswerts Betriebsprogramme mit abnehmender Temperatur der Betriebsprogramme stärker gewichtet sind.

**9.** Waschmaschine nach einem der Ansprüche 2 bis 8, wobei der Hygienerisikowert für ein Betriebsprogramm davon abhängt, was für Waschmittel beim Betriebsprogramm eingesetzt wurden.

**10.** Waschmaschine nach Anspruch 9, wobei der Hygienerisikowert für ein Betriebsprogramm unter Einsatz von Bleichmitteln schwächer gewichtet ist als für das gleiche Betriebsprogramm ohne Einsatz von Bleichmitteln.

**11.** Waschmaschine nach einem der vorangehenden Ansprüche, wobei N nicht grösser als die Zahl der Betriebsprogramme ist, die durchgeführt wurden, nachdem das letzte Mal ein Betriebsprogramm mit einer Temperatur von mindestens 60°C durchlaufen wurde.

**12.** Waschmaschine nach einem der vorangehenden Ansprüche mit einem Bottich und einem ausserhalb des Bottichs angeordneten Dampfgenerator (7), wobei mit dem Dampfgenerator (7) zumindest im Entkeimungsprogramm Dampf erzeugbar und in den Bottich einführbar ist.

**13.** Verfahren zur Steuerung der Waschmaschine mit mehreren unterschiedlichen Betriebsprogrammen,
**dadurch gekennzeichnet, dass**
mindestens einem Teil der Betriebsprogramme mindestens teilweise unterschiedliche Hygienerisikowerte zugeordnet werden,
die Hygienerisikowerte einer Zahl N zuletzt durchgeführter Betriebsprogramme zu einem Hygienebedarfswert H kumuliert werden, wobei die Zahl N mindestens zeitweise grösser als 1 ist, und
abhängig vom Hygienebedarfswert eine Hygieneanzeige (5a der Waschmaschine aktiviert oder deaktiviert wird.

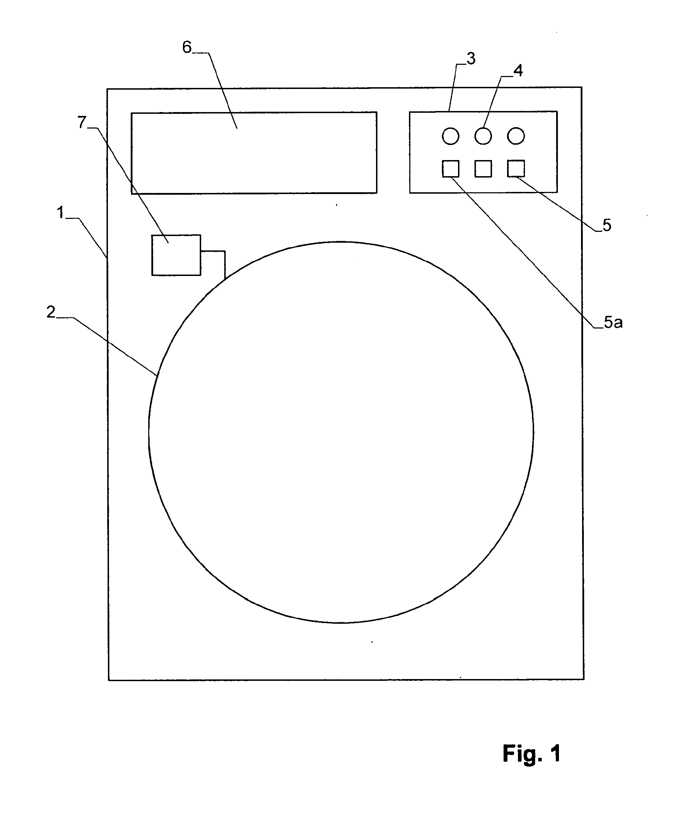

**Fig. 1**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 19751028
- EP 808936 A
- EP 1275767 A